(19) Europäisches Patentamt
European Patent Office
Office européen des brevets

(11) Veröffentlichungsnummer: **0 000 924**
**B1**

# (12) EUROPÄISCHE PATENTSCHRIFT

(45) Veröffentlichungstag der Patentschrift: **29.04.81**

(51) Int. Cl.³: **C 07 D 235/26,** C 07 D 263/58, C 07 D 277/68

(21) Anmeldenummer: **78100686.1**

(22) Anmeldetag: **17.08.78**

## (54) Heterocyclische Phenyläther und Verfahren zu ihrer Herstellung.

(30) Priorität: **30.08.77 DE 2738963**

(43) Veröffentlichungstag der Anmeldung: **07.03.79 Patentblatt 79/5**

(45) Bekanntmachung des Hinweises auf die Patenterteilung: **29.04.81 Patentblatt 81/17**

(84) Benannte Vertragsstaaten: **BE CH DE FR GB NL**

(56) Entgegenhaltungen:
**FR-A-2 110 400**
**NL-A-7 709 744**
**JOURNAL OF THE CHEMICAL SOCIETY**
**1965, Seiten 954—973**
**(In der Anmeldung angeführt)**
*** Seite 960 ***

(73) Patentinhaber: **HOECHST AKTIENGESELLSCHAFT, Postfach 80 03 20, D-6230 Frankfurt/Main 80 (DE)**

(72) Erfinder: **Handte, Reinhard, Dr., Breckenheimer Strasse 45, D-6238 Hofheim am Taunus (DE)**

EP 0 000 924 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

Heterocyclische Phenyläther und Verfahren zu ihrer Herstellung

Aus J. Che. Soc. 1965, 954–73 ist die Verbindung 4-(Benzthiazolyl-2'-oxo)-phenol und ihre Herstellung aus 2-Chlorbenzthiazol und Hydrochinon in siedendem Äthanol in Gegenwart von metallischem Natrium bekannt. Über eine praktische Verwendung dieser Verbindung werden dort keine Angaben gemacht.

Gegenstand der vorliegenden Erfindung sind heterocyclische Phenyläther der allgemeinen Formel I

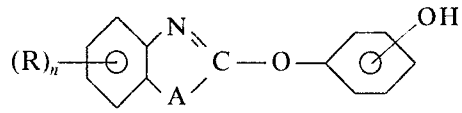 (I)

in der

R gleiche oder verschiedene Reste aus der Gruppe Halogen, $CF_3$, $NO_2$, CN, $(C_1-C_4)$Alkyl, $(C_1-C_4)$Alkoxy, $(C_1-C_4)$Alkylthio,
A O, S oder N-$(C_1-C_4)$alkyl und
n 0 bis 3 bedeuten

mit der Einschränkung, daß für A = S n nicht Null sein kann.

Bevorzugt unter den Verbindungen der Formel I sind solche, in denen n = 0–2 ist, für n ≠ 0 insbesondere solche, in denen R Reste aus der Gruppe Halogen, $CF_3$, $NO_2$, Methyl bedeutet, wobei als Halogen Fluor, Chlor und/oder Brom bevorzugt ist. Ist A = N-Alkyl, so ist N-Methyl bevorzugt.

Gegenstand der Erfindung ist ferner ein Verfahren zur Herstellung der Verbindungen der Formel I, das dadurch gekennzeichnet ist, daß man

a) Verbindungen der Formel II

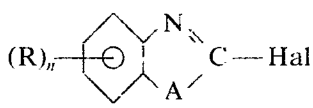 (II)

in der Hal für Halogen, vorzugsweise Cl oder Br steht, oder

b) Verbindungen der Formel IV

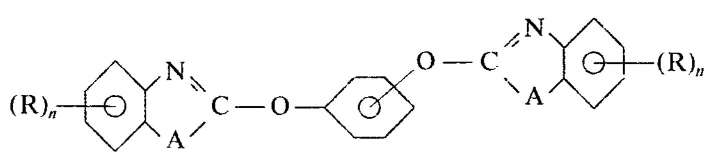 (IV)

mit Verbindungen der Formel III

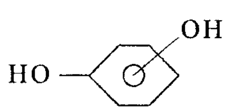

in Gegenwart basischer Verbindungen umsetzt.

Die Verbindungen der Formel II lassen sich nach bekannten Verfahren z. B. aus den entsprechenden 2-Mercapto- bzw. 2-Oxo-Verbindungen durch Halogenierung oder auch aus den 2-Amino-Verbindungen durch Diazotierung und anschließende Sandmeyer-Reaktion herstellen (s. z. B. C. A. 59, 396 j; Am. Chem. J. 21 [1899], 111).

Die Verbindungen der Formel IV lassen sich nach dem Verfahren (a) durch Einsatz von 2 Mol oder mehr der Verbindung II auf 1 Mol der Verbindung III herstellen.

Als Verbindungen der Formel II sind verwendbar die 2-Halogenverbindungen entsprechend substituierter Benzthiazole, Benzoxazole und 1-Alkyl-benzimidazole. Beispiele hierfür sind:

2-Chlor-benzoxazol, -1-methyl-benzimidazol,
2-Chlor-6-fluor-benzthiazol, -benzoxazol, 1-methyl-benzimidazol,

2,6-Dichlor-benzthiazol, -benzoxazol, -1-butyl-benzimidazol,
2,5-Dichlor-benzthiazol, -benzoxazol, -1-methyl-benzimidazol,
2-Chlor-5-methyl-benzthiazol, -benzoxazol, -1-methyl-benzimidazol,
2-Chlor-6-methyl-benzthiazol, -benzoxazol, -1-methyl-benzimidazol,
2-Chlor-6-äthyl-benzthiazol, -benzoxazol, -1-methyl-benzimidazol,
2-Chlor-6-nitro-benzthiazol, -benzoxazol, -1-methyl-benzimidazol,
2,5-Dichlor-6-nitro-benzthiazol, -benzoxazol, -1-methyl-benzimidazol,
2-Chlor-5-methoxy-benzthiazol, -benzoxazol, -1-methyl-benzimidazol,
2-Chlor-6-methoxy-benzthiazol, -benzoxazol, -1-methyl-benzimidazol,
2-Chlor-6-methylthio-benzthiazol, -benzoxazol, -1-methyl-benzimidazol,
2,5,6-Trichlor-benzthiazol, -benzoxazol, -1-methyl-benzimidazol,
2-Chlor-5-brom-benzthiazol, -benzoxazol, -1-methyl-benzimidazol,
2-Chlor-6-brom-benzthiazol, -benzoxazol, -1-methyl-benzimidazol,
2-Chlor-5,6-dibrom-benzthiazol, -benzoxazol, -1-methyl-benzimidazol,
2-Chlor-5-trifluormethyl-benzthiazol, -benzoxazol, -1-methyl-benzimidazol,
2-Chlor-6-trifluormethyl-benzthiazol, -benzoxazol, -1-methyl-benzimidazol,
2-Chlor-6-cyano-benzthiazol, -benzoxazol, -1-methyl-benzimidazol, sowie die entsprechenden 2-Bromderivate.

Die Reaktionstemperaturen liegen zwischen 80° und 250°C, vorzugsweise 90° bis 150°C, gegebenenfalls beim Siedepunkt des verwendeten Lösungsmittels. Bei Temperaturen $<80°$ entsteht bei der Variante a) in zunehmendem Maße die Verbindung IV als an sich unerwünschtes Nebenprodukt. Dieses setzt sich jedoch oberhalb 80° mit weiterem III wieder zu I um (siehe Variante b) ). Auch kann man die Reaktion bei Temperaturen oberhalb der Siedetemperatur im geschlossenen Gefäß unter Eigendruck durchführen; dies ist erforderlich, wenn das verwendete Lösungsmittel unterhalb 80° siedet. Man verwendet vorzugsweise polare aprotische Lösungsmittel, z. B. Säureamide wie Dimethylformamid, Dimethylacetamid, Diäthylacetamid, N-Methylpyrrolidon und Hexamethylphosphorsäuretriamid, ferner Dimethylsulfoxid; Nitrile wie Acetonitril oder Propionitril, Ketone wie Aceton, Methyläthylketon oder Methylisobutylketon. Aber auch aliphatische Alkohole und aromatische Kohlenwasserstoffe wie Toluol oder Xylol sind verwendbar.

Als basische Verbindungen eignen sich die üblichen organischen und anorganischen Basen wie z. B. tertiäre Amine wie Pyridin, Triäthylamin, Alkoholate wie Na-, K-Methylat, -Äthylat, -Butylat, insbesondere jedoch anorganische Basen wie Kalium- oder Natriumhydroxid oder die entsprechenden Carbonate. Sie werden mindestens in stöchiometrischen, vorzugsweise jedoch mindestens äquimolaren Mengen bzw. einem Überschuß von 10–20% darüber eingesetzt. Größere Überschüsse sind möglich, bieten jedoch keinen Vorteil und führen zu einer erhöhten Salzbelastung.

Das Molverhältnis der Reaktanten II : III bzw. IV : III läßt sich in weiten Grenzen variieren. So kann die Verbindung III in doppelt molarer Menge oder mehr bezogen auf II eingesetzt werden. Da jedoch die Entfernung von nicht umgesetztem III zusätzliche Operationen erfordert, die auch zu Ausbeuteverlusten führen können, arbeitet man bevorzugt mit etwa äquimolaren Mengen bzw. einem Überschuß von 5 bis 10% der Verbindung III. Das Arbeiten mit etwa äquimolaren Mengen hat den zusätzlichen Vorteil, daß die Reaktionsprodukte ohne weitere Reinigung weiterverarbeitet werden können.

Das Herstellungsverfahren (a) wird im allgemeinen so durchgeführt, daß man die Verbindung der Formel III mit der basischen Verbindung in einem geeigneten Lösungsmittel zunächst auf Reaktionstemperatur erwärmt, wobei zumindest teilweise das Salz der Verbindung III gebildet wird. Dann gibt man die Verbindung der Formel II, gegebenenfalls gelöst in dem gleichen Lösungsmittel, zu und rührt so lange bei erhöhter Temperatur, bis die Umsetzung beendet ist. Man kann aber auch zunächst das Salz (Monosalz) der Verbindung III vollständig herstellen und dieses mit II umsetzen. Auch ist die Zugabe der Reaktionspartner in anderer Reihenfolge möglich. Die erforderliche Reaktionszeit hängt von der Reaktionstemperatur, vom Lösungsmittel und vom Verhältnis II : III ab und wird geringer mit wachsender Temperatur und wachsendem Überschuß an III. Gewöhnlich ist die Umsetzung nach 30 Minuten bis 50 Stunden beendet.

Das Herstellungsverfahren (b) wird analog (a) durchgeführt, indem statt einer Verbindung II eine Verbindung der Formel IV eingesetzt wird, die wiederum aus II und III erhalten werden kann. Die verwendeten Basen dienen hier zur Herstellung der Salze der Verbindungen III, die sich ihrerseits mit den Bisäthern der Formel IV zu den Verbindungen (Salzen) der Formel I umsetzen.

Beide Reaktionen werden zweckmäßig unter Schutzgas durchgeführt. Als solche sind verwendbar z. B. $N_2$, Ar, $CO_2$, bevorzugt ist $N_2$.

Nach beendeter Reaktion wird das Reaktionsgemisch angesäuert, von gebildetem Salz abfiltriert und — gegebenenfalls nach einer Wasserwäsche — das Lösungsmittel abdestilliert. Bei Arbeiten mit Überschuß an III wird zu seiner Entfernung in einem mit Wasser nicht mischbaren Lösungsmittel — beispielsweise in heißem Xylol — aufgenommen und mit wenig heißem Wasser mehrmals gewaschen, die organische Phase getrocknet und das Lösungsmittel abdestilliert. Andere Aufarbeitungsmethoden sind in den Beispielen beschrieben.

Eine weitere Reinigung der so erhaltenen Verbindungen der Formel I durch Umkristallisieren, Destillieren oder Umfällen ist möglich, doch sind die Produkte vielfach für die Weiterverarbeitung rein genug.

Die Verbindungen der Formel I sind Vorprodukte für wirksame Biozide, z. B. für Herbizide, insbesondere für solche, wie sie in der deutschen Patentanmeldung P 26 40 730.7 vorgeschlagen werden.

Gemäß dieser Anmeldung erhält man durch Umsetzung mit 2-Halogenpropionsäurederivaten wie Estern und Amiden in an sich bekannter Weise aus den Verbindungen der Formel I wertvolle Selektivherbizide. Beispielsweise entsteht aus 4-(6'-Chlorbenzthiazolyl-2'-oxy)-phenol und 2-Brompropionsäureäthylester in Gegenwart von Kaliumcarbonat der 2-[4'-(6''-Chlorbenzthiazolyl-2''-oxy)-phenoxy]-propionsäureäthylester.

Die folgenden Beispiele sollen die Herstellung der erfindungsgemäßen Verbindungen erläutern, ohne sie zu beschränken.

## Herstellungsbeispiele

### Beispiel 1

4-(6'-Chlorbenzthiazolyl-2'-oxy)-phenol

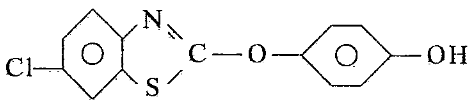

12,1 g (0,11 Mol) Hydrochinon und 16,6 g (0,12 Mol) Kaliumcarbonat werden in 120 ml Dimethylacetamid 1,5 Stunden unter Stickstoffschutzgas bei 95° – 100° C gerührt. Anschließend werden innerhalb von 30 Minuten 20,4 g (0,1 Mol) 2,6-Dichlorbenzthiazol, gelöst in 50 ml Dimethylacetamid, zugetropft. Nach der Zugabe wird die Temperatur bei 95° – 100° C gehalten und der Reaktionsablauf dünnschichtchromatographisch verfolgt. Nach 6 Stunden Reaktionszeit beträgt der Umsatz ca. 90 – 95%. Zur Vervollständigung der Reaktion wird die Temperatur insgesamt 16 Stunden beibehalten. Man filtriert vom Salzanteil ab und rührt das Filtrat in ca. 500 ml Eiswasser ein. Mit 10%iger Schwefelsäure wird anschließend auf pH 2 eingestellt, der helle Niederschlag abgesaugt, mit Wasser neutral gewaschen und getrocknet.

Nach dem Trocknen erhält man 27 g (97,2% d. Th.) 4-(6'-Chlorbenzthiazolyl-2'-oxy)-phenol vom Fp. 174° C. Umkristallisieren aus Xylol ergibt ein Produkt mit Fp. 177° – 178° C.

Zu ähnlichen Ergebnissen gelangt man, wenn man bei sonst gleichen Reaktionsbedingungen an Stelle von Dimethylacetamid N-Methylpyrrolidon, Methyl-isobutylketon, Acetonitril, Methyl-äthylketon, Aceton oder Äthanol (mit Natriumäthylat) anstatt $K_2CO_3$ als Base) verwendet.

### Beispiel 2

4-(5'-Chlorbenzoxazolyl-2'-oxy)-phenol

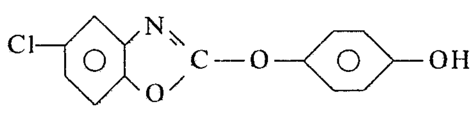

110 g (1 Mol) Hydrochinon und 82,8 g (0,6 Mol) Kaliumcarbonat werden in 600 ml Dimethylformamid 1½ Stunden unter Stickstoffschutzgas bei 120° C gerührt. Innerhalb von 1½ Stunden werden danach 94 g (0,5 Mol) 2,5-Dichlorbenzoxazol in 150 ml Dimethylformamid, gelöst bei 95° C, zugetropft. Nach der Zugabe wird die Temperatur beibehalten, ½ Stunde nach Zugabe ist die Reaktion beendet. Dünnschichtchromatographisch ist nur noch 5'-Chlorbenzoxazolyloxy-phenol und Hydrochinon, jedoch kein 2,5-Dichlorbenzoxazol mehr festzustellen. Der Salzanteil wird abfiltriert und das Filtrat in ca. 2 l Eiswasser eingerührt. Mit 10%iger Schwefelsäure wird auf pH 2 eingestellt und der helle Niederschlag abgesaugt, mit Wasser neutral gewaschen und getrocknet. Nach der Trocknung wird in heißem Xylol aufgenommen und zur Entfernung von überschüssigem Hydrochinon mehrfach mit heißem Wasser gewaschen. Abkühlen der Xylolphase und Absaugen des Niederschlags liefert 106,8 g (81,7% d. Th.) 4-(5'-Chlorbenzoxazolyl-2'-oxy)-phenol mit einem Fp. 179,5° – 181° C.

## Beispiel 3

4-(5′-Chlorbenzthiazolyl-2′-oxy)-phenol

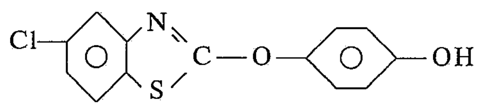

22 g (0,2 Mol) Hydrochinon und 16,6 g (0,12 Mol) Kaliumcarbonat werden in 120 ml Dimethylformamid 1 Stunde bei 120°C unter Stickstoffschutzgas gerührt. Innerhalb von 1 Stunde werden 20,4 g (0,1 Mol) 2,5-Dichlorbenzthiazol, gelöst in 50 ml Dimethylsulfoxid, zugetropft. Nach der Zugabe wird die Temperatur 2 1/2 Stunden beibehalten. Dünnschichtchromatographisch ist danach nur noch 5′-Chlorbenzthiazolyloxy-phenol und Hydrochinon, jedoch kein 2,5-Dichlorbenzthiazol mehr festzustellen. Der Salzanteil wird abfiltriert und das Filtrat in ca. 500 ml Eiswasser eingerührt. Mit 10%iger Schwefelsäure wird auf pH 2 eingestellt und der helle Niederschlag abgesaugt, mit Wasser neutral gewaschen und getrocknet. Nach dem Trocknen wird in heißem Xylol aufgenommen und mehrfach mit heißem Wasser zur Entfernung von überschüssigem Hydrochinon gewaschen. Abkühlen der Xylolphase und Absaugen liefert 23 g (82,7% d. Th.) 4-(5′-Chlorbenzthiazolyl-2′-oxy)-phenol, Fp. 188° – 190° C.

## Beispiel 4

4-(6′-Chlorbenzthiazolyl-2′-oxy)-phenol

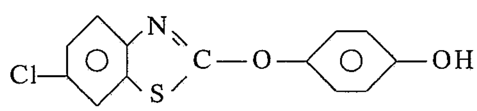

12,1 g (0,11 Mol) Hydrochinon und 16,6 g (0,12 Mol) Kaliumcarbonat werden in 150 ml Dimethylformamid 1 1/2 Stunden unter Stickstoffschutzgas bei 100° C gerührt. Nach der Zugabe von 44,5 g (0,1 Mol) Hydrochinon-1,4-bis-6′-chlor-benzthiazolyl-2′-äther wird die Temperatur beibehalten. Die Spaltung des Bisäthers wird dünnschichtchromatographisch verfolgt. Nach 8 Stunden ist die Reaktion beendet. Nach Abkühlen wird das Reaktionsgemisch in 500 ml Eiswasser eingerührt. Mit 10%iger Schwefelsäure wird auf pH 2 eingestellt und der helle Niederschlag abgesaugt, mit Wasser neutral gewaschen und getrocknet.

Man erhält 50,4 g (91% d. Th.) 4-(6′-Chlorbenzthiazolyl-2′-oxy)-phenol mit Fp. 175° C. Umkristallisieren aus Xylol ergibt ein Produkt mit Fp. 177° – 178° C.

Gemäß den Verfahrensbeispielen 1 bis 4 erhält man ferner:

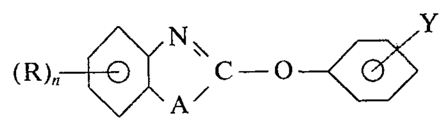

| Beispiel Nr. | $(R)_n$ | A | Y | Fp.(°C) |
|---|---|---|---|---|
| 5 | 5-$CH_3$ | S | 4-OH | 165,5–166 |
| 6 | 6-$CH_3$ | S | 4-OH | 152–152,5 |
| 7 | 6-$NO_2$ | S | 3-OH | 172 |
| 8 | 5-Br | S | 4-OH | 183,5–184 |
| 9 | H | N—$CH_3$ | 4-OH | 203 |
| 10 | H | O | 4-OH | 145–146 |
| 11 | H | N—$CH_3$ | 3-OH | 209 |
| 12 | 5-Cl | O | 4-OH | 179,5–180 |
| 13 | 6-Cl | O | 4-OH | 183,5–184 |
| 14 | H | N—$CH_3$ | 2-OH | 199,5 |
| 15 | 5-Cl 6-$CH_3$ | S | 4-OH | 149–150,5 |
| 16 | 5,6-di-$CH_3$ | S | 4-OH | 171–171,5 |
| 17 | 6-$C_2H_5O$ | S | 4-OH | 139 |
| 18 | 7-Cl | S | 4-OH | 143,5–144 |

**Patentansprüche**

1. Heterocyclische Phenyläther der Formel I

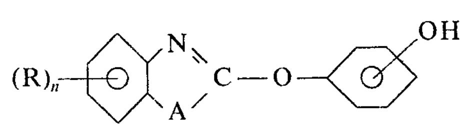

(I)

in der

R gleiche oder verschiedene Reste aus der Gruppe Halogen, $CF_3$, $NO_2$, CN, $(C_1-C_4)$Alkyl, $(C_1-C_4)$Alkoxy, $(C_1-C_4)$Alkylthio,
A O, S oder N-$(C_1-C_4)$alkyl und
n 0 bis 3 bedeuten

mit der Einschränkung, daß für A = S n nicht Null sein kann.

2. Verfahren zur Herstellung von Verbindungen der Formel I, dadurch gekennzeichnet, daß man

a) Verbindungen der Formel II

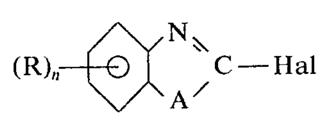

(II)

in der Hal für Halogen, vorzugsweise Cl oder Br steht, oder

b) Verbindungen der Formel IV

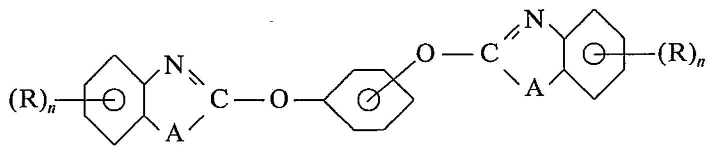

(IV)

mit Verbindungen der Formel III

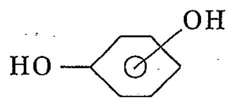

(III)

in Gegenwart basischer Verbindungen umsetzt.

**Claims**

1. Heterocyclic phenyl ethers of the general formula I

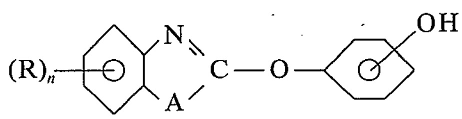

(I)

in which

R are identical or different radicals selected from the group of halogen, $CF_3$, $NO_2$, CN, $(C_1-C_4)$-alkyl, $(C_1-C_4)$alkoxy, $(C_1-C_4)$alkylthio,
A is O, S, or N-$(C_1-C_4)$alkyl and
n is zero to 3,

with the proviso that if A is S, n is other than zero.

2. Process for the manufacture of compounds of formula I, as claimed in claim 1, which comprises reacting

a) compounds of formula II

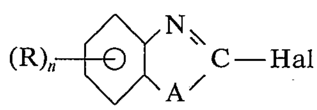

(II)

in which Hal is halogen, preferably chlorine or bromine, or

b) compounds of formula IV

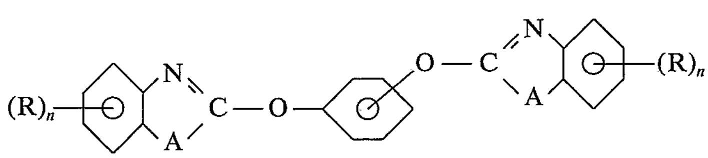

(IV)

with compounds of formula III

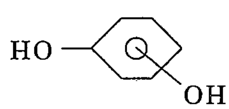

(III)

in the presence of basic compounds.

**Revendications**

1. Ethers phényliques hétérocycliques répondant à la formule I:

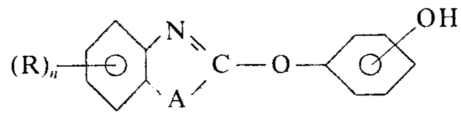

(I)

dans laquelle:

les R représentent des radicaux identiques ou différents pris dans l'ensemble constitué par les halogènes, $CF_3$, $NO_2$, CN, les alkyles en $C_1-C_4$ les alcoxy en $C_1-C_4$ et les alkylthio en $C_1-C_4$;
A représente O, S ou N-($C_1-C_4$)alkyle et
n est un nombre de 0 à 3,

avec la restriction que n ne peut pas être égal à 0 lorsque A représente S.

2. Procédé de préparation de composés de formule I selon la revendication 1, procédé caractérisé en ce qu'on fait réagir:

a) des composés répondant à la formule II:

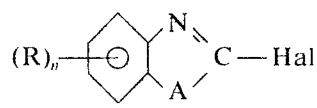

(II)

dans laquelle Hal désigne un halogène, de préférence Cl ou Br, ou

b) des composés de formule IV:

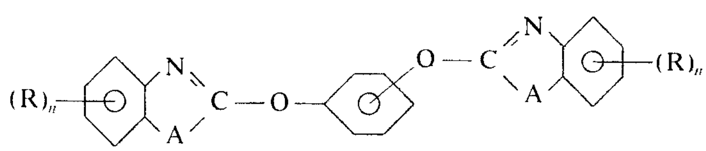

(IV)

avec des composés de formule III:

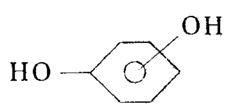

(III)

en présence de composés basiques.